(19) Europäisches Patentamt
European Patent Office
Office européen des brevets

(11) Numéro de publication: **0 010 477**
**B1**

(12) **FASCICULE DE BREVET EUROPEEN**

(45) Date de publication du fascicule de brevet: **15.07.81**

(51) Int. Cl.³: **C 07 D 277/80**

(21) Numéro de dépôt: **79400707.0**

(22) Date de dépôt: **05.10.79**

(54) **Nouveau procédé de préparation des benzothiazole-sulfénamides.**

(30) Priorité: **24.10.78 FR 7830143**

(43) Date de publication de la demande:
**30.04.80 Bulletin 80/9**

(45) Mention de la délivrance du brevet:
**15.07.81 Bulletin 81/28**

(84) Etats Contractants Désignés:
**BE CH DE FR GB IT LU NL SE**

(56) Documents cités:
**FR - A - 2 056 748**
**US - A - 2 782 202**
**US - A - 3 161 648**

(73) Titulaire: **P C U K PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur: **Alicot, Michel Jean Camille**
**Route d'Arreau**
**F-65250 La Barthe de Neste (FR)**
Inventeur: **Ciccotto, Laurent**
**5, rue du Padouen**
**F-65300 Lannemezan (FR)**
Inventeur: **Tignol, Adrien Paul Noel**
**15, rue des Pyrénées**
**F-31210 Montrejeau (FR)**

(74) Mandataire: **Houssin, Jean et al,**
**PRODUITS CHIMIQUES UGINE KUHLMANN**
**Service Propriété Industrielle Tour Manhattan**
**Cedex 21**
**F-92087 Paris La Defense 2 (FR)**

**EP 0 010 477 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveau procédé de préparation des benzothiazole-sulfénamides

La présente invention concerne un nouveau procédé de préparation des benzothiazole-2 sulfénamides, répondant à la formule générale:

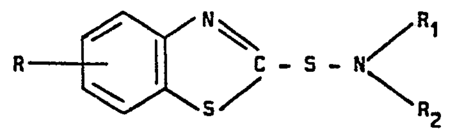

(I)

dans laquelle R représente soit un atome d'hydrogène, soit un groupe alkyle ou nitro, $R_1$ représente soit un atome d'hydrogène, soit un groupe alkyle ramifié ou non, cycloalkyle, alkylcycloalkyle, cyanoalkyle, hydroxyalkyle, $R_2$ représente un groupe alkyle ramifié ou non, cycloalkyle, alkylcycloalkyle, cyanoalkyle, hydroxyalkyle, $R_1$ et $R_2$ peuvent également être des groupes alkylènes reliés entre eux par une liaison simple ou un hétéroatome. Comme exemples de tels groupes, on peut citer les cycles pipéridyle, morpholino.

Les procédés connus concernant la préparation des composés de formule générale (I) utilisent comme matière première de départ, soit le mercaptobenzothiazole, soit le disulfure de benzothiazole.

Dans le premier cas, il est fait appel principalement aux réactions suivantes:

— Condensation oxydante du mercaptobenzothiazole avec une amine en milieu alcalin, généralement en, milieu acueux. L'oxydant est préférentiellement choisi parmi l'hypochlorite de sodium, l'eau oxygénée, le chlore, un persulfate alcalin. Des exemples de ce procédé sont donnés dans le brevet français n° 852.118 déposé le 25 Mars 1939 et le brevet des ETATS—UNIS d'AMERIQUE n° 2.807.620 déposé le 22 Décembre 1955.

— Condensation du mercaptobenzothiazole salifié avec le dérivé N-halogéné d'une amine. Ce procédé est proposé par le brevet français n° 1.165.525 déposé le 14 Novembre 1956 et le brevet des ETATS—UNIS d'AMERIQUE n° 2.730.526 déposé le 21 Mai 1951.

Dans le cas où le disulfure de benzothiazole est la matière première de départ, on peut également avoir recours à une condensation oxydante en présence d'une amine (brevet français n° 852.118 précité). D'une manière générale cependant, les procédés connus mettent en oeuvre la réaction du disulfure de benzothiazole avec une amine et son dérivé N-halogéné. Les brevets des ETATS—UNIS d'AMERIQUE n° 2.730.527 déposé le 12 Décembre 1952, 2.782.202 déposé le 19 Février 1957, 3.022.300 déposé le 23 Septembre 1954, et le brevet anglais n° 856.421 déposé le 27 Mai 1957, sont des illustrations de procédés élaborés selon ce principe.

Ces différents procédés ne sont pas sans inconvénients: les rendements sont parfois insuffisants; la qualité des produits obtenus peut ne pas être tout à fait satisfaisante (en particulier pour de nombreuses applications, la teneur en disulfure de benzothiazole doit être très faible). En outre ces procédés sont souvent spécifiques d'un produit donné et en premettent pas d'obtenir, par exemple, un égal résultat avec une amine primaire ou une amine secondaire. Certains sont exclusifs de l'une ou l'autre de ces deux classes d'amines.

Enfin, un problème difficile à résoudre est celui posé par les rejets, qui peuvent être d'un volume élevé et dont la demande chimique en oxygène peut être très forte: c'est le cas en particulier des procédés en milieu aquaux réalisant la condensation oxydante du mercaptobenzothiazole et d'une amine, dans lesquels un excès important d'oxydant est souvent nécessaire.

Le procédé selon l'invention permet de pallier ces inconvénients. Il est caractérisé en ce que l'on fait réagir un disulfure de benzothiazole de formule générale:

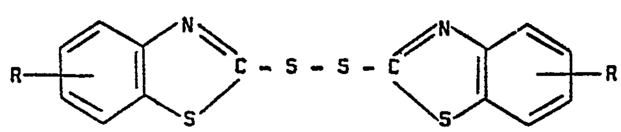

(II)

dans laquelle R a les mêmes significations que ci-dessus avec un mélange d'halogénohydrate d'amine et une N-halogéno amine de formules générales:

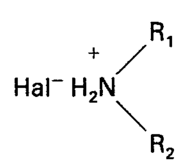 (III) 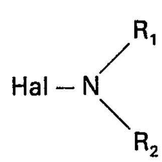 (IV)

dans lesquelles $R_1$ et $R_2$ ont les mêmes significations que ci-dessus.

Pour une mole de disulfure de benzothiazole, on met en oeuvre au moins une mole d'amine sous la forme de dérivé N-halogéné et au moins une mole d'amine sous la forme d'halogénohydrate. On admet préférentiellement un faible excès de 0 à 5% d'équivalent amine par rapport au disulfure de benzothiazole. Il est cependant possible d'engager un excès plus important, lorsque en particulier la récupération de l'excès d'amine peut être réalisée sans difficulté.

L'amine N-halogénée et l'halogénohydrate d'amine sont utilisés dans un rapport stoechiométrique.

La réaction est effectuée dans un milieu solvant miscible à l'eau, préférentiellement choisi dans la classe des alcools aliphatiques, à chaine droite ou ramifiée, tels que les alcools méthylique, éthylique ou isopropylique.

On opère en présence d'un agent alcalin capable de capter l'acide halogéné formé; il est choisi préférentiellement parmi les sels alcalins de l'acide carbonique ou les hydroxydes des métaux alcalins ou alcalino-terreux. Un excès d'agent alcalin n'est pas préjudiciable.

La réaction est conduite dans un intervalle de température compris entre 20°C et la température d'ébullition du solvant choisi, préférentiellement entre 40°C à 70°C.

Bien que l'on puisse réaliser le procédé de l'invention en admettant dans le milieu réactionnel une teneur en eau de l'ordre de 10% en volume, il est préférable de ne pas dépasser la quantité d'eau produite par la réaction de neutralisation de l'acide halogéné par l'agent alcalin utilisé.

La benzothiazole-2 sulfénamide, solubilisée dans le milieu réactionnel au fur et à mesure de sa formation, est précipitée, après éventuelle filtration de l'halogénure alcalin sous-produit, par addition d'eau; elle est isolée par tout moyen connu: filtration, essorage.

Le solvant, séparé de l'eau formée en cours de réaction et de l'eau de dilution, est recyclable. L'eau, dans la plupart des cas, en raison des rendements élevés obtenus, est également totalement ou partiellement recyclable.

Le mélange du dérivé N-halogéné et de l'halogénohydrate de l'amine primaire ou secondaire est préparé au préalable par action d'un halogène sur l'amine correspondante, dans le même milieu solvant que celui utilisé pour la synthèse de la benzothiazole-2 sulfénamide. La réaction peut être représentée comme suit:

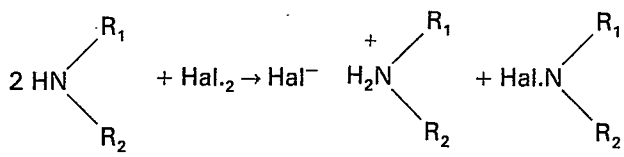

La mesure du potentiel du milieu réactionnel permet de controler aisément le taux de transformation de l'amine et de maitriser la réaction de N-halogénation en toute sécurité, en évitant dans le cas des amines primaires, la formation de dérivés N-dihalogénés qui sont thermiquement instables et capables, à la limite, de se décomposer d'une manière explosive.

La réaction d'halogénation est effectuée à basse température, préférentiellement dans l'intervalle de température −5°C/+10°C de manière à éviter les risques de deshydrohalogénation. Elle est conduite indifféremment en régime continuou discontinu.

Les exemples suivants illustrent l'invention sans la limiter.

Exemple 1

a) Préparation du mélange du chlorhydrate de morpholine et de N-chloro-morpholine.

Dans un réacteur de 750 ml de capacité, muni d'un dispositif d'agitation, d'une prise de température, d'un dispositif d'introduction de gaz, d'un couple d'électrodes verre-alcool méthylique et Hg — $Hg_2Cl_2$ — alcool méthylique, d'une prise de soutirage au voisinage immédiat des électrodes, on introduit pendant 45 minutes de l'alcool méthylique et de la morpholine aux débits respectifs de 475 ml/heure et 122 g/heure, puis, pendant 45 minutes du chlore au débit d'environ 54 g/heure, jusqu'à ce que la valeur du potentiel enregistre entre les deux électrodes soit de + 100 mv (±10 mv). La température, pendant l'introduction du chlore, est régulée à 0°C/+5°C.

Après avoir constitué un volume initial de mélange, on parvient à la:

b) Préparation en continu du mélange,
en procédant simultanément aux opérations suivantes:

— introduction d'alcool méthylique, de morpholine et de chlore, aux débits respectifs de 475 ml/heure, 122 g/heure et 54 g/heure. La température est régulée de à 0°C/+5°C et le débit de chlore est éventuellement ajusté de manière à ce que la valeur du potentiel soit de +100 mv (±10 mv).

— soutirage du produit de réaction à un débit tel que le volume dans le réacteur soit maintenu constant.

La solution est stockée dans un bac maintenu à 0°C/+5°C ou est utilisée directement dans la phase suivante du procédé.

c) Synthèse de la N-(benzothiazyl-2 sulfényl)-morpholine.

Dans un réacteur de 2000 ml de capacité muni d'un dispositif d'agitation, d'une prise de température, on charge sous agitation 700 ml d'alcool méthylique, 332 g de disulfure de benzothiazyle (1 mole) et 120 g de carbonate de sodium.

On chauffe à 50°C/55°C et introduit, en maintenant l'agitation et en 15 minutes environ, 975 ml du mélange préparé sous a) contenant 127,6 g de N-chloromorpholine (soit 1,05 mole) et 129,6 g de chlorhydrate de morpholine (soit 1,05 mole).

La réaction, peu exothermique, est régulée à +50°C/+55°C. Après 1 heure 30, le précipité de chlorure de sodium formé est filtré, lavé avec trois fois 50 ml à 100 ml d'alcool méthylique.

L'ensemble du filtrat et des alcools de lavage est refroidisous agitation à +15°C. La N-(benzothiazyl-2 sulfényl)-morpholine précipite partiellement. On ajoute 2000 ml d'eau froids (entre 10 et 15°C) et maintient sous vive agitation pendant 1 heure 30 à 2 heures. La sulfénamide est totalement insolubilisée. Elle est filtrée, lavée avec environ trois fois 250 ml d'eau, essorée et séché à une température inférieure à 40°C/45°C. On obtient 502 g de N-(benzothiazyl-2 sulfényl)-morpholine de point de fusion (non corrigé) de 85—86°C, de teneur en disulfure de benzothiazyle inférieure à 0,2%. Le rendement, calculé par rapport au disulfure de benzothiazyle, est de 99,55%.

A partir des eaux mères de filtration, et par distillation, on récupère l'alcool méthylique recyclable.

Si l'on dispose de deux installations semblables, de capacité appropriée, travaillant en alternance pour produire la sulfénamide c), la production en continu du mélange b) permet de rendre pseudo-continu l'ensemble du procédé.

Exemple 2

a) Préparation du mélange de chlorhydrate de cyclohexylamine et de N-chlorohexylamine.

Dans un réacteur de 1500 ml de capacité, muni d'un dispositif d'agitation, d'une prise de température, d'une introduction de gaz, d'un couple d'électrodes verre-alcool méthylique et Hg — $Hg_2Cl_2$ — alcool méthylique, d'une prise de soutirage au voisinage immédiat des électrodes, on introduit 900 ml d'alcool méthylique et 208 g de cyclohexylamine (soit 2,10 moles). On ajoute, en maintenant la température à 0°C/+5°C, du chlore gazeux jusqu'à ce que la valeur du potentiel enregistrée entre les deux électrodes soit égale à —100 mv (±10 mv). L'introduction du chlore dure environ une heure quinze minutes et l'on en consomme 80 g. La solution obtenue est maintenue à 0°C/+5°C.

b) Synthèse de la N-(benzothiazyl-2 sulfényl) cyclohexylamine.

Dans un réacteur de 5000 ml, muni d'un dispositif d'agitation et d'une prise de température, on introduit 800 ml d'alcool méthylique, 332 g de disulfure de benzothiazyle (soit 1 mole) et 84 g d'hydroxyde de calcium, et chauffe à 40°C.

On ajoute sous agitation, en 15 minutes environ, la totalité de la solution préparée sous a), contenant 140,2 g de N-monochlorocyclohexylamine et 142,3 g de chlorhydrate de cyclohexylamine (soit 1,05 mole de chacun de ces deux constituants).

La réaction, peu exothermique, est régulée à +40°C. Après 4 heures de contact, on refroidit, toujours sous agitation, à la température de +10°C/+15°C et ajoute 2000 ml d'eau froide (+15°C). La N-(benzothiazyl-2 sulfényl) cyclohexylamine est complètement précipitée après deux heures d'agitation à la température de +15°C; elle est alors filtrée, lavée avec environ 4 à 5 fois 150 ml d'eau (jusqu'à élimination des ions chlorures) et essorée. Après séchage à une température inférieure à 40°C/45°C, on obtient 486 g de sulfénamide, de point de fusion (non corrigé) 98°C/101°C, ayant une teneur en disulfure de benzothiazyle inférieure à 0,4%. Le rendement calculé par rapport au disulfure de benzothiazyle est de 92%.

Exemple 3

a) Préparation du mélange de chlorhydrate de tertiobutylamine et de N-chlorotertiobutylamine.

Dans un réacteur de 1500 ml de capacité, muni d'un dispositif d'agitation, d'une prise de température, d'une introduction de gaz, d'un couple délectrodes verre-alcool méthylique et Hg — $Hg_2Cl_2$ — alcool méthylique d'une prise de soutirage au voisinage immédiat des électrodes, on introduit 900 ml d'alcool méthylique et 182,5 g de tertio-butylamine (soit 2,5 moles). En maintenant la température entre 0 et 5°C, on introduit du chlore gazeux jusqu'à ce que la valeur du potentiel entre les deux électrodes soit égale à +10 mv (±10 mv). On absorbe ainsi en une heure, environ 98 g de chlore.

b) Synthèse de la N-(benzothiazyl-2 sulfényl) t.butylamine.

Dans un réacteur annexe de 5000 ml de capacité muni d'une agitation, d'une prise de température, on met en suspension 332,5 g de disulfure de benzothiazyle (soit une mole) et 144 g de carbonate de sodium, dans 500 ml d'alcool méthylique.

On ajoute, toujours sous agitation, et en 20 minutes environ, la solution préparée en a) contenant 134 g de N-monochloro-t.butylamine et 136,5 g de chlorhydrate de t.butylamine. On chauffe une heure à 60—65°C. Après refroidissement à 10—15°C, on ajoute 1500 ml d'eau et agite trois heures à 15—20°C. La sulfénamide précipitée est filtrée, lavée à l'eau jusqu'à élimination des ions chlorures, essorée et séchée à une température inférieure à 40—45°C.

On obtient 453 g de sulfénamide de point de fusion (non corrigé): 107°C et dont la teneur en disulfure de benzothiazyle est inférieure à 0,4%. Le rendement par rapport au disulfure de benzothiazyle est de 95%.

## Revendications

1. Procédé pour la preparation des benzothiazole-sulfénamides de formule générale:

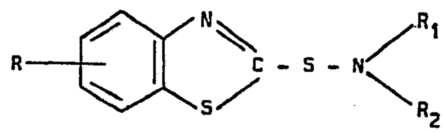

(I)

dans laquelle R représente soit un atome d'hydrogène, soit un groupe alkyle ou nitro, $R_1$ représente soit un atome d'hydrogène, soit un groupe alkyle ramifié ou non, cycloalkyle, alkylcycloalkyle, cyanoalkyle, hydroxyalkyle, $R_2$ représente un groupe alkyle ramifié ou non, cycloalkyle, alkylcycloalkyle, cyanoalkyle, hydroxyalkyle, $R_1$ et $R_2$ peuvent également être des groupes alkylènes reliés entre eux par une liaison simple ou un hétéroatome, caractérisé en ce qu'on fait réagir un disulfure de benzothiazyle de formule générale:

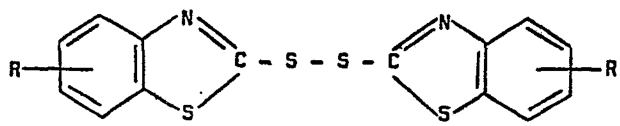

(II)

avec un mélange comprenant un halogénohydrate d'amine de formule générale:

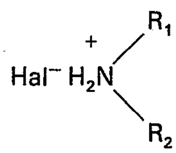

et une N-halogéno-amine de formule générale:

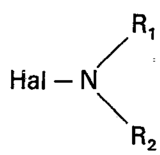

R, $R_1$ et $R_2$ ayant les mêmes significations que ci-dessus.

2. Procédé tel que revendiqué sous 1) dans lequel on met en oeuvre au moins une mole d'halogénohydrate et au moins une mole de N-halogéno-amine pour une mole de disulfure.

3. Procédé tel que revendiqué sous 1) dans lequel l'halogénohydrate et la N-halogénoamine sont dans un rapport stoechiométrique.

4. Procédé tel que revendiqué sous 1) dans lequel la réaction est effectuée dans un solvant miscible à l'eau.

5. Procédé tel que revendiqué sous 1) dans lequel la réaction est effectuée en présence d'un agent alcalin.

6. Procédé tel que revendiqué sous chacune des revendications 1 et 4 dans lequel la réaction est effectuée entre 20°C et la température d'ébullition du solvant.

7. Procédé tel que revendiqué sous chacune des revendications 1 et 3 dans lequel le mélange de l'halogénohydrate et de la N-halogéno-amine est préparé à l'avance par action de l'halogène $Hal._2$ sur une amine de formule générale:

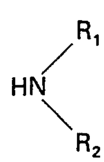

dans le même solvant que celui utilisé pour la synthèse de la sulfénamide, la température étant de préférence inférieure à 10°C.

**Claims**

1. Process for the preparation of benzothiazole-sulphenamides of the general formula:

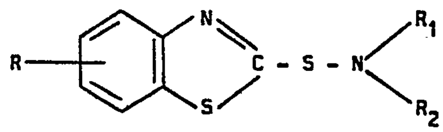

(I)

in which R represents either a hydrogen atom or an alkyl or nitro group, $R_1$ represents either a hydrogen atom or a branched or unbranched alkyl, a cyclcalkyl, alkylcycloalkyl, cyanoalkyl or hydroxyalkyl group, $R_2$ represents a branched or unbranched alkyl, a cycloalkyl, alkylcycloalkyl, cyanoalkyl or hydroxyalkyl group, and $R_1$ and $R_2$ may also be alkylene groups interconnected by means of a single bond or a hetero atom, characterised in that a benzothiazyl disulphide of the general formula:

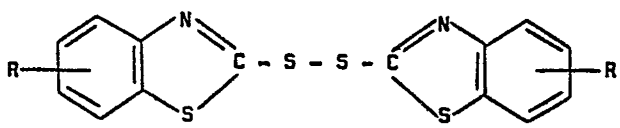

(II)

is reacted with a mixture comprising an amine halogenohydrate of the general formula:

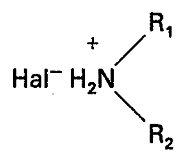

and an N-halogeno-amine of the general formula:

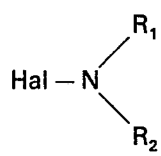

R, $R_1$ and $R_2$ having the same meanings as above.

2. Process as claimed in claim 1, in which at least one mole of halogenohydrate and at least one mole of N-halogeno-amine are used per one mole of disulphide.

3. Process as claimed in claim 1, in which the halogenohydrate and the N-halogen-amine are in a stoichiometric ratio.

4. Process as claimed in claim 1, in which the reaction is effected in a solvent which is miscible with water.

5. Process as claimed in claim 1, in which the reaction is effected in the presence of an alkaline agent.

6. Process as claimed in each of claims 1 and 4, in which the reaction is effected between 20°C and the boiling temperature of the solvent.

7. Process as claimed in each of claims 1 and 3, in which the mixture of halogenohydrate and N-halogeno-amine is prepared in advance by the action of the halogen $Hal._2$ on an amine of the general formula:

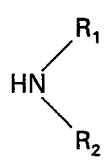

in the same solvent as that used for the synthesis of the sulphenamide, the temperature being preferably below 10°C.

## Patentansprüche

1. Verfahren zur Herstellung von Benzthiazolsulfenamiden der allgemeinen Formel:

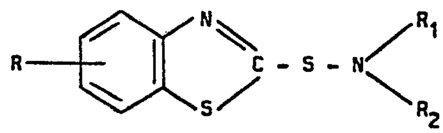

in der R entweder ein Wasserstoffatom, eine Alkyl- oder Nitrogruppe, $R_1$ entweder ein Wasserstoffatom, eine verzweigte oder geradkettige Alkylgruppe, eine Cycloalkyl-, Alkylcycloalkyl-, Cyanoalkyl- oder Hydroxyalkylgruppe bedeuten, $R_1$ und $R_2$ auch durch eine Einfachbindung oder ein Heteroatom miteinander verbundene Alkylengruppen sein können, dadurch gekennzeichnet, daß man ein Benzthiazoldisulfide der allgemeinen Formel:

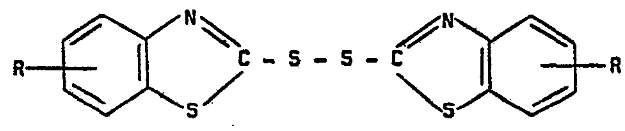

mit einem Gemisch aus einem Halogenhydrat eines Amins der allgemeinen Formel:

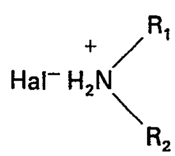

und einem N-halogenamin der allgemeinen Formel

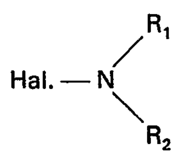

wobei R, $R_1$ und $R_2$ die oben angegebenen Bedeutungen aufweisen, zur Umsetzung bringt.

2. Verfahren nach Anspruch 1, bei dem man mindestens ein Mol Halogenhydrat und mindestens ein Mol N-Halogenamin je Mol Disulfid einsetzt.

3. Verfahren nach Anspruch 1, bei dem das Halogenhydrat und das N-Halogenamin in einem stöchiometrischen Verhältnis vorliegen.

4. Verfahren nach Anspruch 1, bei dem die Umsetzung in einem mit Wasser mischbaren Lösungsmittel durchgeführt wird.

5. Verfahren nach Anspruch 1, bei dem die Umsetzung in Gegenwart eines alkalischen Mittels durchgeführt wird.

6. Verfahren nach einem der Ansprüche 1 und 4, bei dem die Umsetzung zwischen 20°C und dem Kochpunkt des Lösungsmittels durchgeführt wird.

7. Verfahren nach einem der Ansprüche 1 und 3, bei dem das Gemisch des Halogenhydrates und des N-Halogenamins vorab durch Einwirkung des Halogens $Hal_2$ auf ein Amin der allgemeinen Formel hergestellt wird:

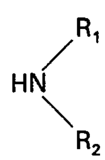

im gleichen Lösungsmittel, wie dies für die Herstellung des Sulfenamids verwendet wird, wobei die Temperatur vorzugsweise unterhalb 10°C liegt.